# EUROPÄISCHE PATENTSCHRIFT

(11) **EP 0 281 090 B1**
(45) Veröffentlichungstag und Bekanntmachung des Hinweises auf die Patenterteilung: **19.05.1993**
(21) Anmeldenummer: 88103141.3
(22) Anmeldetag: 02.03.1988
(51) Int. Cl.: C12N 15/12, C12P 21/02, A61K 37/64

(54) **Tendamistat-Derivate**
Tendamistate derivatives
Dérivés de tendamistal

(30) Priorität: 06.03.1987 DE 3707150
(43) Veröffentlichungstag der Anmeldung: 07.09.1988
(73) Patentinhaber: HOECHST AKTIENGESELLSCHAFT, 65926 Frankfurt am Main (DE)
(72) Erfinder: Koller, Klaus-Peter, Dr., D-6232 Bad Soden am Taunus (DE); Engels, Joachim, Prof. Dr., D-6242 Kronberg/Taunus (DE); Neeb, Martin, Dr., D-6000 Frankfurt am Main (DE)

(56) Entgegenhaltungen:
- EP-A- 0 161 629
- DE-A- 3 331 860
- BIOLOGICAL CHEMISTRY HOPPE SEYLER, Band 368, Nr. 9, September 1987, Seite 1039, DE; J.W. ENGELS et al.: "Sits specific mutagenesis on the alpha-amylase inhibitor "Tendamistat""
- JOURNAL OF MOLECULAR BIOLOGY, Band 189, Nr. 2, 1986, Seiten 383-386, GB; J.W. PFLUGRATH et al.: "Crystal structure determination, refinement and the molecular model of the alpha-amylase inhibitor Hoe-467A

## Beschreibung

Die Erfindung betrifft Proteine, die sich von dem α-Amylase-Inhibitor Tendamistat ableiten.

Die für Tendamistat kodierende DNA ist in der veröffentlichten europäischen Patentanmeldung (EP-A 1) 0 161 629 bzw. in der südafrikanischen Patentschrift 85/3672 (als DNA-Sequenz C) angegeben. In diesem Strukturgen befinden sich mehrere Schnittstellen für Restriktionsenzyme, die zur Modifikation der kodierten Aminosäurefolge genutzt werden können. Geeignet sind die Schnittstellen für BstEII im Bereich der Tripletts 31 und 32, StuI im Bereich der Tripletts 43 und 44 sowie Sau3A im Bereich der Tripletts 52 und 53. Durch Einbau entsprechender Linker können an diesen Stellen eine oder mehrere zusätzliche Aminosäuren eingeschoben werden, DNA-Segmente zwischen diesen Schnittstellen eliminiert werden oder durch Einbau von Stopp-Kodons verkürzte Aminosäuresequenzen kodiert werden. Darüber hinaus können durch ortsspezifische Mutagenese beliebige Aminosäuren eingefügt, ausgetauscht oder eliminiert werden. Man erhält so Proteine, die α-Amylase-Inhibitorwirkung zeigen, oder aber Proteine ohne diese Wirksamkeit, die aber noch mit den entsprechenden Rezeptoren reagieren.

Die erfindungsgemäßen Tendamistat-Derivate stellen also einerseits modifizierte α-Amylase-Inhibitoren dar. Durch die unterschiedliche Struktur sind folgende Veränderungen in den Eigenschaften möglich: Durch eine reduzierte Halbwertszeit nach der Applikation ist eine bessere Kontrolle des Hemmprozesses möglich. Eine verbesserte Abbaubarkeit durch Proteasen in der Blutbahn verkürzt die Verweildauer im Blut. Nicht durch α-Amylase gebundene Inhibitoren, die beispielsweise durch Phagocytose in die Blutbahn gelangen, rufen dort keine oder eine stark verringerte Antikörperbildung hervor, weil immunogene Regionen im Molekül beseitigt oder verändert wurden. Durch diese verringerte Immunogenität wird eine Langzeittherapie mit diesen Substanzen ermöglicht.

Andererseits können die Tendamistat-Derivate, insbesondere diejenigen mit deutlich verkürzter Aminosäurekette, in reversibler Weise mit den spezifischen Rezeptoren in Form eines kompetitiven Hemmechanismus reagieren.

Eine weitere Möglichkeit für eine erfindungsgemäße Modifikation des Tendamistatgens besteht darin, das Tendamistat-Strukturgen als "Träger" für ein anderes Gen zu benutzen. Man erhält so Fusionsproteine, die aus der Wirtszelle sekretiert werden können und nach Isolierung aus dem Kulturmedium chemisch oder enzymatisch gespalten werden.

Die Erfindung betrifft außerdem Plasmide, die die modifizierten Gene enthalten und insbesondere Expressionsplasmide, die befähigt sind, die kodierten Tendamistat-Derivate in Streptomyceten zu exprimieren.

Die Expression der modifizierten Inhibitoren kann in an sich bekannter Weise erfolgen, wobei zweckmäßig das dem natürlichen Tendamistatgen vorgelagerte Signalpeptid zur Sekretion der Inhibitoren in das Kulturmedium genutzt wird. Die Isolierung der exprimierten und sezernierten Proteine kann ebenfalls in an sich bekannter Weise, vorteilhaft durch Adsorptions- oder Ionenaustauschchromatographie und/oder Gelfiltration erfolgen.

In den folgenden Beispielen wird die Erfindung näher erläutert. Sofern keine anderen Angaben gemacht sind, beziehen sich Prozentangaben auf das Gewicht.

Als Ausgangsplasmid dient pKAI650, dessen Herstellung in der nicht vorveröffentlichten europäischen Patentanmeldung EP-A2 0 218 204 bzw. in der entsprechenden ZA-A 86/7697 vorgeschlagen ist. Zur Herstellung dieses Plasmids wird aus in dem in der deutschen Offenlegungsschrift 3 331 860 beschriebenen Plasmid pKAI1 ein 650bp HincII-SstI-Fragment isoliert und in das handelsübliche Plasmid pUC19 eingesetzt, das zuvor mit diesen Enzymen geöffnet wurde.

### Beispiel 1

2 µg über CsCl-Gradientenzentrifugation gereinigte DNA von pKAI650 wird 2 Stunden in einem 50 µl-Reaktionsansatz mit StuI nach den Angaben des Herstellers komplett verdaut und das Enzym durch Phenolextraktion entfernt. Die linearisierte DNA wird mit Ethanol gefällt, wieder gelöst und in ein Ligationsgemisch eingebracht, dem als zusätzlicher Reaktionspartner 0,1 µg des chemisch synthetisierten, am 5ʹ-Ende phosphorylierten, doppelsträngigen Oligonukleotids (1) 
BamHI
5' C G C G G A T C C G C G 3' (1)
3' G C G C C T A G G C G C 5'
zugegeben wird.

Nach Transformation des Ligationsgemisches in E. coli JM 109 werden solche Klone isoliert, die das rekombinante Plasmid pKK1 tragen. Die isolierte Plasmid-DNA ist durch eine zusätzliche Schnittstelle für das Restriktionsenzym BamHI gekennzeichnet, die eine Charakterisierung durch Restriktionsanalyse erlaubt. pKK1 ist um 12 Basenpaare größer als pKAI650 und weist eine Nukleotidsequenz auf, durch die die Aminosäuresequenz wie folgt um 4 Aminosäuren erweitert wird: 
42 43 (44)
Glu Gly Ala Asp Pro Arg Leu
5' GAA GG C GCG GAT CCG CG C CTG 3'
3' CTT CC G CGC CTA GGC GC G GAC 5'
Isolierte pKK1-Plasmid-DNA wird nach Doppelverdauung mit den Restriktionsendonukleasen SstI und SphI einer präparativen Gelelektrophorese unterworfen und das ca. 660 bp umfassende Fragment mit dem modifizierten Amylase-Inhibitor (AI)-Gen isoliert.

### Beispiel 2

Verfährt man entsprechend Beispiel 1, setzt jedoch anstelle des Oligonukleotids (1) das synthetische Oligonukleotid (2) 
BamHI
5' C C C G G A T C C G G C 3'
3' G G G C C T A G G C C G 5' (2)
ein, so erhält man das Plasmid pKK2. Dieses kodiert für einen modifizierten AI mit der folgenden veränderten Aminosäurefolge:

42      43                                    (44)
Glu     Gly     Pro     Asp     Pro     Gly     Leu

GAA     GG C    CCG     GAT     CCG     GC C    CTG
CTT     CC G    GGC     CTA     GGC     CG G    GAC

### Beispiel 3

In die singuläre StuI-Schnittstelle von pKAI650 wird analog den Beispielen 1 und 2 folgende 12mer-Nukleotidsequenz (3) eingefügt: 
HindIII
5' C C C A A G C T T G G G 3' (3)
3' G G G T T C G A A C C C 5'
Das nach Transformation von E. coli JM 109 erhaltene Plasmid pKK3 ist durch eine zusätzliche Schnittstelle für das Restriktionsenzym HindIII gekennzeichnet, 12 Basenpaare größer als pKAI650 und weist eine Nukleotidsequenz auf, durch welche die Aminosäuresequenz wie folgt um 4 Aminosäuren erweitert wird: 
42 43 (44)
Glu Gly Pro Ser Leu Gly Leu
5' GAA GG C CCA AGC TTG GG C CTG 3'
3' CTT CC G GGT TCG AAC CC G GAC 5'
In Analogie zu Beispiel 1 wird das ca. 660 bp umfassende Fragment mit dem modifizierten AI-Gen isoliert.

### Beispiel 4

Verfährt man gemäß Beispiel 1, setzt jedoch anstelle des Oligonukleotids (1) das chemisch synthetisierte und an den 5ʹ-Enden phosphorylierte Oligonukleotid (4) 
5' C ATG AAG CAC TCC GAC GGT ACC TTC ACC TCC GAG CTG TCC CGC CTC
3' G TAC TTC GTG AGG CTG CCA TGG AAG TGG AGG CTC GAC AGG GCG GAG
(4)
GCG CTG AGG CGG GCG GAG GTC GCC GAC GAG GTC CCG GAG CAG CCG TTC CC 3'
CGC GAC TCC GCC CGC CTC CAG CGG CTG CTC CAG GGC CTC GTC GGC AAG GG 5'
ein, so erhält man das um 96 Basenpaare vergrößerte Plasmid pKK4. Dieses kodiert für einen modifizierten AI mit der folgenden veränderten Aminosäuresequenz: 
42  43              1*                                  10*
Glu Gly Met Lys His Ser Asp Gly Thr Phe Thr Ser Glu Leu Ser Arg Leu
5' GAA GGC ATG AAG CAC TCC GAC GGT ACC TTC ACC TCC GAG CTG TCC CGC CTC
3' CTT CCG TAC TTC GTG AGG CTG CCA TGG AGG TGG AGG CTC GAC AGG GCG GAG
 
20*                                   28*     (44)
Arg Asp Ser Ala Arg Leu Gln Arg Leu Leu Gln Gly Leu Val Gly Lys Gly Leu
CGC GAC TCC GCC CGC CTC CAG CGG CTG CTC CAG GGC CTC GTC GGC AAG GGC CTG 3'
GCG CTG AGG CGG GCG GAG GTC GCC GAC GAG GTC CCG GAG CAG CCG TTC CCG GAC 5'
In Analogie zum Beispiel 1 wird das ca. 750 bp umfassende SstI-SphI-Fragment mit dem modifizierten AI-Gen isoliert und gemäß Beispiel 8 und 9 zur Expression gebracht.

Durch Einwirkung der Endoproteinase Lys-N (AL-1 Protease II, Wingard et al., J. Bacteriology 112 (1972) 940-949) aus Lysobacter enzymogenes, die das Protein an der aminoterminalen Seite von Lysin spaltet, sowie durch Inkubation z.B. mit der Endoproteinase Lys-C (US-Patentschrift 4 414 332) aus Lysobacter enzymogenes, die das Protein an der carboxyterminalen Seite von Lysin spaltet, auf den aus dem Kulturfiltrat isolierten Inhibitor, erhält man ein 28 Aminosäuren umfassendes Protein, das chromatographisch gereinigt werden kann und das nach chemischer oder enzymatischer Amidierung am Gly²⁸* funktionell dem Hormon Sekretin entspricht.

Ein entsprechendes Protein erhält man durch Einwirkung z.B. der Endoproteinase Lys-C und anschließender Einwirkung des Enzyms Carboxypeptidase B. Durch dieses Vorgehen läßt sich der modifizierte Inhibitor für die Synthese eines funktionalen Fremdeiweißes nutzen.

### Beispiel 5

1 µg isolierter Plasmid-DNA von pKAI650 wird vollständig mit dem Restriktionsenzym BstEII verdaut. Das Enzym wird durch Phenolextraktion entfernt, die linearisierte DNA mit Ethanol gefällt und mit 0,1 µg der chemisch synthetisierten und am 5ʹ-Ende phosphorylierten DNA (5) folgender Struktur ligiert: 
5' GTG ACC GAG TAA TGA AAT TC 3'
3' G CTC ATT ACT TTA AGC ACTG 5' (5)
Nach Transformation in E. coli JM 109 werden solche Klone isoliert, die 2 Schnittstellen für das Enzym EcoRI aufweisen und das gesuchte Plasmid pKK5 tragen. pKK5 kodiert für einen Inhibitor, der folgende carboxyterminale Struktur aufweist:
Glu²⁹ - Thr³⁰ - Val³¹ - Thr³² - Glu³³ - COOH
Der Inhibitor ist folglich gegenüber Tendamistat um 41 Aminosäuren verkürzt und wirkt im Gegensatz zu diesem nicht mehr irreversibel, sondern weist einen kompetitiven Hemmechanismus auf.

### Beispiel 6

Zur Eliminierung der Cysteine in Position 11 und 27 nach der "gapped duplex"-Methode (B. Kramer et al., Cell 38 (1984) 879-889) werden die folgenden einsträngigen Oligonukleotide nach der β-Cyanethoxyphosphoramidit-Methode (N.D. Sinha et al., Tetrahedron Letters 24 (1983) 5843) mit dem DNA-Synthesizer 380A der Firma Applied Biosystems, Pfungstadt, hergestellt. Die Ansatzgröße ist 0,2 µmol, die Ausbeute pro Syntheseschritt 96,9 %.

7 8 9 10 (11) 12 13 14 15 16
Pro Ala Pro Ser Ala Val Thr Leu Tyr Gln
5' G CCC GCA CCC TCC GCG GTG ACG CTC TAC CAG AG 3' (6)
SstII

24 25 26 (27) 28 29 30
Asp Asn Gly Ala Ala Glu Thr
5' C GAC AAC GGC GCT GCC GAG ACG G 3' (7)

Diese Oligonukleotide (6) und (7) werden als mutagene "Primer" eingesetzt, was zur Folge hat, daß die beiden genannten Cysteine durch Alanin ersetzt werden.

Die synthetisierten Oligonukleotide werden über präparative Polyacrylamid-Gelelektrophorese getrennt und die Produktbanden ausgeschnitten. Die Oligonukleotide werden aus dem Gel eluiert und das Eluat durch Chromatographie gereinigt (^{R}SEPHADEX G50 fein und BIOGEL P6, 100 bis 200 mesh, Säule 25 · 1,6 cm). Die Ausbeuten betragen 4,6 nMol für das Oligonukleotid der Formel (7) und 11,7 nMol für das Oligonukleotid der Formel (6).

Das 650 bp große PstI-SstI-Fragment aus pKAI650 wird in den Phagen M13mp18am umkloniert (M13mp18am enthält zwei amber-Mutationen in essentiellen Genen). Mit den rekombinanten Phagen werden E. coli-Zellen (BMH 71-18), einer Suppressor-Mutante, transfiziert und auf Xgal-IPTG-Platten ausplattiert. Farblose Einzelplaques werden ausgestochen, die Phagen in BMH 71-18 kultiviert und die DNA aus verschiedenen Klonen isoliert. Die positiven Klone enthalten das 650 bp-Fragment aus pKAI650.

Die ssDNA (+-Strang) aus den rekombinanten Phagen wurde mit dem größeren der beiden Fragmente aus der PstI-SstI-Verdauung des Phagen M13mp18rev inkubiert; hierbei entstand die "gapped duplex DNA" mit den beiden Ambermutationen und der Insertion auf dem +-Strang und dem "gap" auf dem revertanten --Strang. Dann wird die "gapped duplex"-DNA mit den Primern (7) und (8) inkubiert, die DNA zum Doppelstrang aufgefüllt, ligiert und damit E. coli BMH 71-18mut S transfiziert. Die mutS-Mutante von E. coli kann keine "mismatch"-Reparatur durchführen und ermöglicht damit die Vermehrung von Phagen mit der genetischen Konstitution sowohl des +- als auch des --Stranges.

Die anschließend in E. coli MK 30-3 kultivierten und durch Restriktionsanalyse als positiv identifizierten Klone werden sequenziert, wobei alle 8 getesteten Klone das 650 bp SstI-HindIII-Fragment mit der AI-Sequenz enthalten und 5 dieser 8 Klone die neu engeführte SstII-Schnittstelle aufweisen. Der Stamm E. coli MK 30-3 ist keine Suppressor-Mutante, so daß sich lediglich Phagen mit der genetischen Konstitution des --Stranges (rev) vermehren können.

Aus der RF-DNA der Phagen, die das modifizierte AI-Gen tragen, wurde das SstI-SphI-Insert in das Plasmid pUC19 umkloniert, wobei die Plasmide pKAI750 und pKAI850 erhalten werden. Die Isolierung des SstI-SphI-Inserts, das das veränderte AI-Gen trägt, erfolgt gemäß Beispiel 1.

### Beispiel 7

Das 650 bp große SstI-SphI-DNA-Fragment aus pKAI650 wird gemäß Beispiel 6 in den E. coli-Phagen M13mp11 umkloniert, die Einzelstrang-DNA isoliert und diese mit Hilfe des mutagenen "Primer"
3ʹ C TCG ACC GAC ATG AGT G 5ʹ
und des Mutagenese-Systems der Firma Amersham (Braunschweig, Code Nr. NPM 2322) nach der Eckstein-Methode mutagenisiert. Es resultiert ein doppelstrangiges DNA-Fragment, in dem, wie durch DNA-Sequenzierung belegt, im kodierenden Bereich für Tendamistat das Codon CGG für Arg¹⁹ gegen das Codon CTG für Leu¹⁹ ausgetauscht wurde. Das so mutagenisierte SstI-SphI-Fragment wird analog Beispiel 6 in pUC19 umkloniert, wobei das Plasmid pKAI 950 erhalten wird. Durch Expression des mutierten Gens erhält man ein Tendamistat-Derivat mit veränderten Bindungseigenschaften, das als kompetitiver Inhibitor wirkt.

### Beispiel 8

Das Plasmid pIJ702 wird mit den Restriktionsendonukleasen SphI und SstI komplett verdaut und das ca. 400 bp SstI-SphI-Fragment aus der Tyrosinase-Gen-Region (Hopwood, D.A. et al., Genetic Manipulation of Streptomyces: A Laboratory Manual John Innes Foundation, Norwich, 1985) von der verbleibenden Vektor-DNA durch Gelelektrophorese in einem 0,5 %igen Agarosegel abgetrennt. 1 µg der so gereinigten Vektor-DNA wird mit 0,2 µg des gemäß Beispiel 1 bis 7 isolierten SstI-SphI-Fragments mit dem modifizierten Inhibitorgen ligiert. Das Ligations-Gemisch wird in an sich bekannter Weise in Streptomyces lividans TK 24 transformiert. Die Selektion der gewünschten Klone erfolgt auf Resistenz gegenüber Thiostrepton (20 µg/ml) und Produktion von Amylase-Inhibitor gemäß dem nachstehend beschriebenen Plattentest

### Plattentest:

Die Kolonien werden mit 5 ml einer 0,4 bis 1,0 mg/ml Pankreatin enthaltenden wäßrigen Lösung übergossen und 1 Stunde bei 37°C inkubiert. Die Lösung wird dann entfernt und durch 5 ml eines 2 Gew.-%igen Stärke-Agars ersetzt. Nach 2 Stunden Inkubation bei 37°C werden die Platten mit 5 ml einer Jod-Kaliumjodid-Lösung zur Entwicklung übergossen. Kolonien mit einem blauen Hof zeigen an, daß die Klone AI synthetisieren und exkretieren.

### Beispiel 9

Transformierte Klone von S. lividans werden auf Nährmedium R2YE (Hopwood et al., a. a. O.) mit 20 µg/ml Thiostrepton übertragen und durch Bebrütung bei 26 bis 28°C für 5 bis 7 Tage zur Versporung gebracht.

Ca. 0,25 cm² versporten Mycels werden in 100 ml Erlenmeyer-Kolben überimpft, die mit 25 ml des folgenden Kulturmediums gefüllt sind:

| | |
|---|---|
| Sojamehl | 2 % |
| Glucose | 3 % |
| Maisstärke | 0,2 % |
| Harnstoff | 0,1 % |
| Ammoniumcitrat | 0,1 % |
| Malzextrakt | 0,5 % |
| KH₂PO₄ | 0,2 % |

Das Medium wird 30 Minuten bei 121°C und 1 Bar autoklaviert, der pH-Wert beträgt 7,0 bis 7,2. Die Impfkolben werden 2 Tage bei 28°C auf einer Schüttelmaschine inkubiert. Dann werden 2 ml der gewachsenen Vorkultur in 20 ml Hauptkulturmedium, das sich in 100 ml Erlenmeyer-Kolben befindet, überimpft:

| | |
|---|---|
| lösliche Stärke | 2 - 4 % |
| Sojamehl | 0,4 % |
| Cornsteep, flüssig | 0,4 % |
| Magermilchpulver | 0,7 % |
| Glucose | 1,0 % |
| (NH₄)₂HPO₄ | 1,2 % |

Der pH-Wert des Mediums beträgt nach dem Autoklavieren pH 7,6.

Die Kultur wird für 4 Tage bei 28°C und 220 U/min geschüttelt. Danach wird das Mycel von der Kulturlösung durch Zentrifugation abgetrennt und der sich in der Kulturlösung befindliche Inhibitor isoliert.

## Patentansprüche (Patentansprüche für folgende(n) Vertragsstaat(en): AT, BE, CH, DE, FR, GB, IT, LI, LU, NL, SE)

1. Tendamistat-Derivate, deren Aminosäuresequenz sich von Tendamistat durch Austausch, Einschub oder Eliminierung von Aminosäuren unterscheidet.

2. Tendamistat-Derivate nach Anspruch 1, die innerhalb der Tendamistat-Aminosäuresequenz die Aminosäuresequenz eines anderen Polypeptids oder Proteins enthalten.

3. Verfahren zur Herstellung der Tendamistat-Derivate nach Anspruch 1 oder 2, dadurch gekennzeichnet, daß das Strukturgen für Tendamistat durch Basenaustausch, Eliminierung oder Zufügung von Basen variiert und das derart veränderte Gen exprimiert wird.

4. Plasmide, enthaltend ein Gen, das für ein Tendamistat-Derivat nach Anspruch 1 oder 2 kodiert.

5. Arzneimittel, bestehend aus einem Tendamistat-Derivat nach Anspruch 1.

6. Arzneimittel, enthaltend ein Tendamistat-Derivat nach Anspruch 1 und einen pharmakologisch akzeptablen Träger.

7. Verwendung der Tendamistat-Derivate nach Anspruch 2 zur Freisetzung des darin enthaltenen Polypeptids oder Proteins.

8. Verfahren zur Exkretion von Tendamistat-Derivaten nach Anspruch 2 aus Streptomyceten, dadurch gekennzeichnet, daß man die kodierende Sequenz für das gewünschte Protein in das gegebenenfalls modifizierte Tendamistatgen einbaut und das rekombinante Gen in einer Streptomycetenzelle als Fusionsprotein exprimiert.

## Patentansprüche (Patentansprüche für folgende(n) Vertragsstaat(en): ES , GR)

1. Verfahren zur Herstellung von Tendamistat-Derivaten, deren Aminosäuresequenz sich von Tendamistat durch Austausch, Einschub oder Eliminierung von Aminosäuren unterscheidet, dadurch gekennzeichnet, daß das Strukturgen für Tendamistat durch Basenaustausch, Eliminierung oder Zufügung von Basen variiert und das derart veränderte Gen exprimiert wird.

2. Verfahren nach Anspruch 1, dadurch gekennzeichnet, daß in das Strukturgen für Tendamistat das Gen für ein anderes Polypeptid oder Protein eingebaut wird und das entsprechende Fusionsprotein exprimiert wird.

3. Verfahren nach Anspruch 1 oder 2, dadurch gekennzeichnet, daß man das Gen, das für ein Tendamistat-Derivat nach Anspruch 1 oder 2 kodiert, in ein Plasmid einbaut und damit die Wirtszelle transformiert.

4. Verfahren zur Herstellung von Tendamistat-Derivaten nach Anspruch 2, dadurch gekennzeichnet, daß man die kodierende Sequenz für das gewünschte Protein in das gegebenenfalls modifizierte Tendamistatgen einbaut, das rekombinante Gen in einer Streptomycetenzelle exprimiert und das Fusionsprotein aus dem Kulturmedium gewinnt.

5. Verwendung der nach Anspruch 1, 2, 3 oder 4 erhältlichen Tendamistat-Derivate zur Freisetzung eines darin enthaltenen Proteins.

## Claims (Claims for the following Contracting State(s): AT, BE, CH, DE, FR, GB, IT, LI, LU, NL, SE)

1. A tendamistat derivative whose amino acid sequence differs from that of tendamistat owing to replacement, insertion or deletion of amino acids.

2. A tendamistat derivative as claimed in claim 1 which contains within the tendamistat amino acid sequence the amino acid sequence of another polypeptide or protein.

3. A process for the preparation of the tendamistat derivatives as claimed in claim 1 or 2, which comprises altering the structural gene for tendamistat by base exchange, or deletion or addition of bases, and expressing the gene which has been altered in this way.

4. A plasmid containing a gene which codes for a tendamistat derivative as claimed in claim 1 or 2.

5. A pharmaceutical composed of a tendamistat derivative as claimed in claim 1.

6. A pharmaceutical containing a tendamistat derivative as claimed in claim 1 and a pharmacologically acceptable vehicle.

7. The use of the tendamistat derivatives as claimed in claim 2 for liberating the protein or polypeptide contained therein.

8. A process for the excretion of tendamistat derivatives as claimed in claim 2 from Streptomyces, which comprises incorporating the coding sequence for the desired protein into the tendamistat gene, which has been modified where appropriate, and expressing the recombinant gene in a Streptomyces cell as fusion protein.

## Claims (Claims for the following Contracting State(s): ES, GR)

1. A process for the preparation of tendamistat derivatives whose amino acid sequence differs from that of tendamistat owing to replacement, insertion or deletion of amino acids which comprises altering the structural gene for tendamistat by base exchange, or deletion or addition of bases, and expressing the gene which has been altered in this way.

2. The process as claimed in claim 1, wherein the gene for another polypeptide or protein is incorporated into the structural gene for tendamistat, and the corresponding fusion protein is expressed.

3. The process as claimed in claim 1 or 2, wherein the gene which codes for a tendamistat derivative as claimed in claim 1 or 2 is incorporated into a plasmid, and the host cell is transformed therewith.

4. A process for the preparation of tendamistat derivatives as claimed in claim 2, which comprises incorporating the coding sequence for the required protein into the optionally modified tendamistat gene, expressing the recombinant gene in a Streptomyces cell, and obtaining the fusion protein from the culture medium.

5. The use of the tendamistat derivatives obtainable as claimed in claim 1, 2, 3 or 4 for liberating a protein contained therein.

## Revendications (Revendications pour l'(les) Etat(s) contractant(s) suivant(s): AT, BE, CH, DE, FR, GB, IT, LI, LU, NL, SE)

1. Dérivés de tendamistat, dont la séquence d'aminoacides diffère de celle du tendamistat par remplacement, insertion ou élimination d'aminoacides.

2. Dérivés de tendamistat selon la revendication 1, qui contiennent, au sein de la séquence d'aminoacides du tendamistat, la séquence d'aminoacides d'un autre polypeptide ou d'une protéine.

3. Procédé pour la production des dérivés de tendamistat selon la revendication 1 ou 2, caractérisé en ce que le gène structural codant pour le tendamistat est modifié par remplacement de bases, élimination ou introduction de bases, et le gène modifié de cette façon est exprimé.

4. Plasmides contenant un gène qui code pour un dérivé de tendamistat selon la revendication 1 ou 2.

5. Médicament constitué d'un dérivé de tendamistat selon la revendication 1.

6. Médicament contenant un dérivé de tendamistat selon la revendication 1 et un véhicule pharmacologiquement acceptable.

7. Utilisation des dérivés de tendamistat selon la revendication 2, pour la libération de la protéine ou du polypeptide contenu dans ceux-ci.

8. Procédé pour l'excrétion de dérivés de tendamistat selon la revendication 2, par des Streptomyces, caractérisé en ce que l'on incorpore dans le gène du tendamistat, éventuellement modifié, la séquence codante pour la protéine recherchée, et le gène recombinant est exprimé, sous forme d'une protéine de fusion, dans une cellule de Sreptomyces.

## Revendications (Revendications pour l'(les) Etat(s) contractant(s) suivant(s): ES, GR)

1. Procédé pour la production de dérivés de tendamistat dont la séquence d'aminoacides diffère de celle du tendamistat par remplacement, insertion ou élimination d'aminoacides, caractérisé en ce que le gène structural codant pour le tendamistat est modifié par remplacement de bases, élimination ou introduction de bases, et le gène modifié de cette façon est exprimé.

2. Procédé selon la revendication 1, caractérisé en ce que l'on incorpore dans le gène structural codant pour le tendamistat le gène codant pour un autre polypeptide ou une protéine, et la protéine de fusion correspondante est exprimée.

3. Procédé selon la revendication 1 ou 2, caractérisé en ce que l'on incorpore dans un plasmide le gène qui code pour un dérivé de tendamistat selon la revendication 1 ou 2, et on transforme avec celui-ci la cellule hôte.

4. Procédé pour la production de dérivés de tendamistat selon la revendication 2, caractérisé en ce que l'on incorpore dans le gène structural, éventuellement modifié, la séquence codante pour la protéine recherchée, le gène recombinant est exprimé dans une cellule de Streptomyces et on recueille la protéine de fusion à partir du milieu de culture.

5. Utilisation des dérivés de tendamistat pouvant être obtenus selon la revendication 1, 2, 3 ou 4, pour la libération d'une protéine contenue dans ceux-ci.
